# FASCICULE DE BREVET EUROPEEN

(11) **EP 2 758 449 B1**
(45) Date de publication et mention de la délivrance du brevet: **16.01.2019**
(21) Numéro de dépôt: 12759162.6
(22) Date de dépôt: 18.09.2012
(51) Int. Cl.: C08G 73/10, C08J 3/12, C08L 79/08

(54) **(CO)POLYIMIDES THERMOPLASTIQUES ET PROCEDES DE SYNTHESE**
THERMOPLASTISCHE (CO)POLYIMIDE UND SYNTHESEVERFAHREN DAFÜR
THERMOPLASTIC (CO)POLYIMIDES AND SYNTHESIS METHODS

(30) Priorité: 20.09.2011 FR 1158321; 20.09.2011 FR 1158326; 23.02.2012 FR 1251644
(43) Date de publication de la demande: 30.07.2014
(73) Titulaire: Rhodia Operations, 93306 Aubervilliers (FR)
(72) Inventeur: JEOL, Stéphane, F-69006 Lyon (FR)
(74) Mandataire: Vande Gucht, Anne
(86) Numéro de dépôt international: PCT/EP2012/068367
(87) Numéro de publication internationale: WO 2013/041530

(56) Documents cités:
- US-A- 2 867 609
- US-A- 4 251 417
- US-A- 5 104 966
- US-A- 5 413 888
- US-A- 5 922 167
- US-A- 6 066 710
- US-A1- 2011 065 257

## Description

La présente invention concerne de nouveaux (co)polyimides thermoplastiques et leurs procédés de synthèse. Plus précisément, la présente invention vise des (co)polyimides thermoplastiques, semi-aromatiques obtenus par polymérisation d'au moins un composé aromatique comprenant 2 fonctions anhydride et/ou ses dérivés acide carboxylique et/ou ester, et au moins une diamine aliphatique particulière. Ces (co)polyimides peuvent être transformés en articles plastiques par diverses méthodes telles que l'extrusion, le moulage ou le soufflage.

### ART ANTERIEUR

Les polyimides, notamment les polyimides aromatiques, sont connus pour leurs propriétés thermiques et/ou mécaniques remarquables, ce qui les destine en particulier à des applications hautes performances dans différents domaines tel que l'aéronautique ou encore l'électronique (cartes de circuits imprimés par exemple).

Néanmoins, ces polyimides aromatiques semi-cristallins présentent un certain nombre d'inconvénients. Ils sont généralement infusibles, c'est à dire qu'ils se dégradent avant de fondre (à plus de 500°C), et considérés comme des thermodurs, c'est-à-dire qu'une fois formés on ne peut pas les mettre en oeuvre par refusion. Leurs températures de mise en oeuvre sont généralement trop élevées pour permettre une transformation par les procédés de mise en oeuvre classiques des polymères thermoplastiques, comme les polyamides, notamment l'extrusion, le soufflage ou le moulage. Par ailleurs, une mise en oeuvre à de telles température peut entraîner des dégradations significatives de la matrice polyimide et/ou des phénomènes de colorations préjudiciables pour la réalisation de pièces « esthétiques ». Au surplus, leurs températures de fusion élevées gênent ou empêchent l'utilisation de certains additifs comme des agents ignifugeants organophosphorés ou des fibres naturelles qui se décomposent à de telles températures. Un autre inconvénient des polyimides aromatiques vient des diamines aromatiques qui sont pour la plupart cancérigènes.
En raison des points évoqués ci-dessus, et en particulier des difficultés rencontrées pour réaliser des pièces en polyimides aromatiques, les utilisateurs se tournent fréquemment vers d'autres polymères de hautes performances comme le PEEK (PolyEtherEtherKétone), un polymère semi-cristallin de température de fusion de 340°C et de température de transition vitreuse, Tg, de 150°C, qui est donc compatible avec les procédés de transformation des thermoplastiques par refusion.

Les polyimides aromatiques peuvent être fusibles, donc transformables par les techniques d'extrusion ou de moulage, lorsqu'elles comprennent des diamines aromatiques ou des dianhydrides plus flexibles. Par exemple de tels polyimides amorphes de température de transition vitreuse Tg proche de 200°C sont connus sous le nom de polyétherimide dont Ultem est un nom commercial.

Sont également connus des copolyimides semi-aromatiques obtenus par polymérisation d'acide tétracarboxylique aromatique avec un mélange de diamine aromatique et de diamine aliphatique, comme décrits dans les brevets US5922167 et US5104966. La teneur en diamine aliphatique dans le mélange de départ n'excède toutefois pas 50%. Cette condition permettant de conserver la rigidité du polymère, la diamine aromatique ayant pour effet d'apporter de la rigidité au copolyimide obtenu, ce qui permet de balancer l'effet des diamines aliphatiques, qui assouplissent le polymère en faisant baisser la valeur de la température de transition vitreuse. Ces copolyimides sont amorphes, l'utilisation de deux types de diamine perturbant la cristallinité du polymère. En outre, l'utilisation de diamines aromatiques susceptibles de présenter des effets cancérigènes n'est pas satisfaisante.

En outre, des polyimides semi-aromatiques obtenus par polymérisation d'acide tétracarboxylique aromatique et de diamine aliphatique sont connus de US2710853, qui décrit des diamines aliphatiques particulières à chaîne longue, les deux fonctions amines étant séparées par au moins 7 atomes de carbone. Si ces polyimides présentent une aptitude à la mise en oeuvre intéressante, étant donné qu'ils peuvent être mis en forme à 360°C, en revanche leur température de transition vitreuse est trop basse (environ 135°C) pour rivaliser avec d'autres polymères hautes performances, comme par exemple le PEEK de Tg=150°C et de Tf=340°C.

Les polyimides semi-aromatiques constituent donc une approche intéressante aux problèmes posés car ils peuvent être semi-cristallins et/ou être transformables par les procédés de mise en oeuvre connus pour les thermoplastiques, de par leurs températures de fusion compatibles avec les températures de transformation des polymères thermoplastiques, c'est-à-dire une température de fusion généralement inférieure à 350°C, tout en bénéficiant d'une excellente tenue en température.

Cependant, les polyimides semi-aromatiques existants à ce jour ne sont pas suffisamment performants pour rivaliser avec les polymères thermoplastiques classiquement utilisés dans le domaine des polymères de hautes performances comme le PEEK, possédant à la fois une Tf élevée mais compatible avec les outils de mise en oeuvre et une Tg très élevée à 150°C, mais dont certains de leurs monomères présentent une toxicité importante et/ou dont les procédés de synthèse peuvent se révéler insatisfaisants en terme de respect de l'environnement, notamment du fait de l'utilisation de monomères toxiques.

L'objectif de la présente invention est donc de trouver de nouveaux polymères ayant de bonnes propriétés thermiques, mécaniques, diélectriques (isolantes), ainsi qu'une bonne stabilité dimensionnelle. De plus, les polymères devront présenter une Tg élevée. Avantageusement, les polymères seront semi-cristallins avec des températures de fusion compatibles avec les températures de transformation des polymères thermoplastiques usuels et de haute performances, une température de fusion généralement inférieure à 350°C, et donc étant transformables par les procédés de mise en oeuvre connus pour les thermoplastiques (extrusion, moulage, soufflage). En particulier, les polymères pourront être obtenus à partir d'une grande variété de monomères, présentant une toxicité faible, voire nulle, éco-amicaux, peu onéreux, largement disponibles et/ou faciles à synthétiser...

### INVENTION

Il vient d'être mis en évidence par la demanderesse des (co)polyimides particuliers, thermoplastiques et semi-aromatiques qui satisfont en tout ou partie les objectifs précités. Ces (co)polyimides peuvent être préparés en utilisant comme monomères constitutifs des diamines spécifiques décrites ci-après.

La présente invention concerne ainsi un (co)polyimide thermoplastique, semi-aromatique obtenu par polymérisation d'unités récurrentes qui sont constituées par les composés suivants :
(a) au moins un composé aromatique comprenant 2 fonctions anhydride et/ou ses dérivés acide carboxylique et/ou ester, choisi dans le groupe constitué par : l'anhydride pyromellitique, le 3,3',4,4'-biphényltétracarboxylique dianhydride, l'acide pyromellitique, l'acide 3,3',4,4'-biphényltétracarboxylique, et leurs mélanges ; et
(b) une ou plusieurs diamines aliphatiques dont les fonctions amines ne sont pas liées de manière covalente à un atome de carbone d'un cycle aromatique,
dans lequel ladite ou lesdites diamines aliphatiques sont choisies parmi les diamines de formule (I) NH₂-R-NH₂ avec R étant un radical divalent hydrocarboné aliphatique saturé, dont les deux fonctions amines sont séparées par 4 à 6 atomes de carbone, et dont 1 ou 2 atomes d'hydrogène du radical divalent sont substitués par 1 ou 2 groupes méthyle et/ou éthyle; le (co)polyimide pouvant comprendre en outre comme autre diamine aliphatique (b) une ou plusieurs diamines de formule (II) NH₂-R'-NH₂ avec R' étant un radical divalent hydrocarboné, aliphatique, cycloaliphatique, ou arylaliphatique, saturé ou insaturé, qui comprend éventuellement des hétéroatomes;
ou au moins un sel de carboxylate d'ammonium obtenu à partir des monomères (a) et (b).

On comprend donc, au sens de la présente invention, que les unités récurrentes dudit (co)polyimide sont constituées par les composés (a) et (b), dans lesquelles les diamines (b) sont telles que définies par la formule (I) et éventuellement la formule (II).

On peut donc reformuler l'invention d'une autre manière : un (co)polyimide thermoplastique, semi-aromatique obtenu par polymérisation des composés suivants :
(a) au moins un composé aromatique comprenant 2 fonctions anhydride et/ou ses dérivés acide carboxylique et/ou ester ; et
(b) une ou plusieurs diamines aliphatiques
dans lequel au moins une diamine aliphatique est choisie parmi les diamines de formule (I) NH₂-R-NH₂ avec R étant un radical divalent hydrocarboné aliphatique saturé, dont les deux fonctions amines sont séparées par 4 à 6 atomes de carbone, et dont 1 ou 2 atomes d'hydrogène du radical divalent sont substitués par 1 ou 2 groupes méthyle et/ou éthyle.
ou au moins un sel de carboxylate d'ammonium obtenu à partir des monomères (a) et (b).

Le (co)polyimide de l'invention peut comprendre en outre comme autre diamine aliphatique (b) une ou plusieurs diamines de formule (II) NH₂-R'-NH₂ avec R' étant un radical divalent hydrocarboné, aliphatique, cycloaliphatique, ou arylaliphatique, saturé ou insaturé, qui comprend éventuellement des hétéroatomes.

La présente invention concerne aussi un sel de carboxylate d'ammonium obtenu à partir d'un ou plusieurs composés (a) et d'une ou plusieurs diamines aliphatiques (b) telles que définies ci-dessus ou plus bas dans la description.

L'invention concerne aussi des procédés de préparation des (co)polyimides selon l'invention par polymérisation des monomères (a) et (b) ou du sel de carboxylate d'ammonium obtenu à partir des monomères (a) et (b).

L'invention concerne également des particules solides de (co)polyimide à base de (co)polyimide selon l'invention dont le diamètre médian D50 est inférieur ou égal à 20 mm, de préférence compris entre 2µm et 10 mm.

L'invention vise également des compositions comprenant le (co)polyimide de l'invention et des charges et/ou additifs.

L'invention vise aussi un procédé de fabrication d'article plastique mettant en oeuvre des particules solides de (co)polyimide selon l'invention, notamment par refusion desdites particules puis mise en forme d'un article, notamment par extrusion, moulage ou soufflage.

Enfin, l'invention concerne donc un article plastique obtenu à partir du (co)polyimide selon l'invention ou de la composition le comprenant. Il peut s'agir d'une pièce moulée comme par exemple une pièce moulée par injection ou un composite à fibres continues, une pièce extrudée comme par exemple un film, une fibre, un fil, ou un filament, ou encore une pièce soufflée. Il peut aussi s'agir d'une pièce tissée ou tricotée à partir de fibres, fils ou filaments à base de (co)polyimide selon l'invention.

### Définitions

Par « diamine aliphatique », on entend au sens de la présente invention qu'il s'agit de diamines dont les fonctions amines ne sont pas liées de manière covalente à un atome de carbone d'un cycle aromatique comme un phényle par exemple. Par contre si la fonction amine est liée à un groupement benzyle, celle-ci est couverte par le terme « aliphatique », en effet il s'agit alors d'une amine arylaliphatique.

On entend par « semi-cristallin », un (co)polyimide présentant une phase amorphe et une phase cristalline, ayant par exemple un taux de cristallinité compris entre 1% et 85%. On entend par «amorphe» un (co)polyimide ne présentant pas de phase cristalline détectée par les analyses thermiques (type DSC « Differential Scanning Calorimetry »).

On entend par « (co)polyimide thermoplastique », un (co)polyimide présentant une température au-delà de laquelle la matière se ramollit et fond et qui, au dessous de celle-ci, devient dure.

On entend par « diamètre médian D50 », la médiane qui sépare en deux parties d'aires égales la courbe de répartition granulométrique en volume. Les analyses granulométriques peuvent être effectuées au moyen d'un granulomètre à diffraction laser Mastersizer X à banc optique étendu de la Société Malvern Instruments SA permettant de caractériser des tailles de particules comprises entre 2 et 2000 µm. Pour les particules de taille comprise entre 100 µm et 20 mm, les mesures sont effectuées par microscopie optique, en mesurant la plus grande longueur de 100 particules prises au hasard dans un échantillon de particules de (co)polyimide. La distribution étant volumique, le diamètre médian correspondra à 50% du volume total des particules. De plus, le diamètre médian donné, correspond au diamètre d'une sphère équivalente ; en supposant que tous les objets ont une forme équivalente à une sphère.

Lorsque l'on prépare un polyimide à partir d'un sel de carboxylate d'ammonium, la détermination de la température de fusion du sel est préférentiellement effectuée par la mesure de la température de fin de l'endotherme mesurée par calorimétrie différentielle à balayage (DSC « Differential Scanning Calorimetry »), à l'aide d'un appareil Perkin Elmer Pyris 1, en chauffant le sel à partir de 20°C à une vitesse de 10°C/min.

La détermination de la température de fusion du (co)polyimide est préférentiellement effectuée au pic de l'endotherme de fusion mesurée par calorimétrie différentielle à balayage (DSC « Differential Scanning Calorimetry »), à l'aide d'un appareil Perkin Elmer Pyris 1, en chauffant le (co)polyimide à partir de 20°C à une vitesse de 10°C/min.

On entend par particules selon la présente invention des objets pouvant prendre diverses formes telles que des formes sphériques, sensiblement sphériques, quasi-sphériques, polyédriques, ovoïdes et/ou ellipsoïdales et pouvant présenter en surface des aspérités ou de petites cavités formant des irrégularités, générés par des bulles de gaz par exemple. Les particules peuvent être des microbilles, des perles, des agrégats, des granules, des agglomérats, des grains, de la poudre ou autres. On préfère notamment les poudres, dont le diamètre médian D50 peut généralement aller de 2 µm à 2000 µm. Les granulés, dont le diamètre médian D50 peut généralement aller de 2 mm à 5 mm, sont également particulièrement avantageux selon l'invention.

La présente invention concerne des (co)polyimides obtenus à partir d'un ou plusieurs acides tétracarboxyliques aromatiques et une ou plusieurs diamines aliphatiques spécifiques répondant à la définition de l'invention. Les polymères obtenus à partir d'une seule diamine et d'un seul acide tétracarboxylique sont des polyimides, généralement appelés homopolyimides. La réaction entre au moins 3 monomères différents produit un copolyimide. Les (co)polyimides peuvent être définis par la composition molaire en chaque monomère constitutif.

### Les (co)polyimides

Les (co)polyimides de l'invention présentent avantageusement une température de transition vitreuse Tg supérieure à 100°C, en particulier supérieure à 150°C.
Lorsqu'un polymère présente une Tg supérieure à 100°C, cela signifie que lorsque les articles faits avec ce polymère sont utilisés à une température inférieure à 100°C, le polymère est dans son état vitreux, c'est-à-dire l'état dans lequel il est le plus rigide. Il y a un grand nombre d'applications dans lesquelles la température d'utilisation de ces articles n'excède pas 100°C et plus particulièrement 150°C : par exemple un habitacle dans l'automobile, les bâtiments, ... Le dimensionnement d'une pièce est effectué à la température à laquelle l'article va être utilisé. Ainsi, si on utilise un polymère dont la Tg est supérieure à la température d'utilisation, les calculs prennent en compte la rigidité élevée du polymère. Par rapport à un polymère dont la Tg serait inférieure à la température d'utilisation, on peut donc se permettre le cas échéant d'utiliser moins de matière.
Tout particulièrement, les (co)polyimides de l'invention présentent une Tg inférieure ou égale à 250°C. Ceci peut notamment permettre une mise en oeuvre plus facile.
Les (co)polyimides selon l'invention peuvent être semi-cristallins et présenter ainsi des températures de fusion compatibles avec les températures de transformation des polymères thermoplastiques usuels et de hautes performances. Les (co)polyimides selon l'invention peuvent ainsi présenter une température de fusion Tf allant de 100 à 350°C, en particulier de 150°C à 350°C. Avantageusement, ces (co)polyimides possèdent des températures de cristallisation élevées permettant de réduire significativement les temps de cycle de production.

Les polymères amorphes présentent l'avantage d'être transparents (lorsqu'il ne sont pas formulés), ce qui est important en optique. Pour pouvoir être utilisé, ces polymères doivent impérativement présenter une Tg supérieure à la température d'utilisation.
Les polymères semi-cristallins présentent l'avantage de conserver des propriétés mécaniques au-delà de leur Tg, jusqu'à leur Tf.

Les (co)polyimides obtenus sont des thermoplastiques et ont comme propriété de ne pas, ou peu, libérer ou absorber d'eau lors d'étapes ultérieures de transformation, comme la pultrusion, l'extrusion ou le moulage par injection. Ces (co)polyimides peuvent présenter ainsi une excellente stabilité dimensionnelle.

Les (co)polyimides selon l'invention peuvent avoir une masse volumique réelle (c'est à dire celle du matériau non poreux) supérieure ou égale à 0,9 g/cm³, de préférence supérieure ou égale à 1 g/cm³. Cette masse volumique peut notamment être déterminée par le rapport entre la masse exprimée en gramme et le volume exprimé en cm³ d'une pièce pleine en (co)polyimide selon l'invention, de forme parallépipède rectangle et de dimension Longueur(L) x largeur(I) x épaisseur(e). La mesure de la masse volumique est effectuée à température ambiante, typiquement à 23°C.

### Monomères

### Les composés (a)

Les composés (a) portent préférentiellement des fonctions acides carboxyliques dans des positions telles qu'elles permettent de former deux fonctions anhydrides d'acides sur une même molécule (par une réaction de déshydratation). Les composés (a) présentent généralement deux paires de fonctions acides carboxyliques, chaque paire de fonction étant liée à un atome carbone adjacent, en α et β.
Les fonctions acides tétracarboxyliques peuvent être obtenues à partir de dianhydrides d'acides par hydrolyse des fonctions anhydrides. Des exemples de dianhydrides d'acides et d'acide tétracarboxyliques, dérivés des dianhydrides, sont décrits dans le brevet US7932012.

Les composés (a) de l'invention peuvent également porter au moins un autre groupe fonctionnel. Ce groupe peut notamment être choisi parmi le groupe - SO₃X, avec X=H ou un cation, notamment Na, Li, Zn, Ag, Ca, Al, K et Mg, le groupe hydroxyle -OH, le groupe cétone C=O, et les éthers -O-.

Les composés aromatiques comprenant 2 fonctions anhydride sont choisis parmi l'anhydride pyromellitique, le 3,3',4,4'-biphényltétracarboxylique dianhydride, et leurs mélanges. Ces composés, en combinaison avec les diamines (b) de l'invention, présentent l'avantage de donner des (co)polyimides thermoplastiques, semi-cristallins et dont la Tg est supérieure à 150°C.

Parmi les dianhydrides cités ci-dessus, l'anhydride pyromellitique est particulièrement intéressant, notamment car il est facile à mettre en oeuvre, peu onéreux, largement disponible et facile à synthétiser.

Les composés aromatiques comprenant des fonctions acides carboxyliques, notamment dérivés des 2 fonctions anhydride sont choisis parmi l'acide pyromellitique, l'acide 3,3',4,4'-biphényltétracarboxylique, et leurs mélanges. Ces composés, en combinaison avec les diamines (b) de l'invention, présentent l'avantage de donner des (co)polyimides thermoplastiques, semi-cristallins et dont la Tg est supérieure à 150°C.

L'acide pyromellitique est particulièrement intéressant.

Alternativement, les composés (a) de l'invention peuvent être les esters des dianhydrides ou des tétraacides obtenus par réaction du dianhydride ou du tétraacide avec un monoalcool tel que le méthanol, l'éthanol, le propanol et isomères, le butanol et leurs isomères. Il peut s'agir de monoesters, de diesters (ou hemiesters), de triesters ou de tétraesters. On préfère les diesters, et en particulier le diester de l'acide pyromellitique.

Dans un mode particulièrement préféré de réalisation de l'invention, les composés (a) sont des dianhydrides ou des acides tétracarboxyliques puisque ces composés présentent l'avantage de ne pas dégager de produits secondaires de réaction autres que de l'eau, et en particulier ils ne dégagent pas de solvants tels que des alcools. En particulier, on préfère l'anhydride pyromellitique ou l'acide pyromellitique.
De façon avantageuse, ces composés (a) préférés (l'anhydride pyromellitique ou l'acide pyromellitique) représentent au moins 80% molaire par rapport à l'ensemble des composés (a) mis en oeuvre.

### Les composés (b)

Selon l'invention, le (co)polyimide a pour monomère constitutif au moins une diamine aliphatique dont les fonctions amines ne sont pas liées de manière covalente à un atome de carbone d'un cycle aromatique, de formule (I) NH₂-R-NH₂ avec R étant un radical divalent hydrocarboné aliphatique saturé, dont les deux fonctions amines sont séparées par 4 à 6 atomes de carbone, et dont 1 ou 2 atomes d'hydrogène du radical divalent sont substitués par 1 ou 2 groupes méthyle et/ou éthyle.

Il s'agit de diamines de chaîne relativement courte et ramifiée, qui de façon surprenante, en combinaison avec l'acide ou l'anhydride pyromellitique, l'acide ou l'anhydride 3,3',4,4'-biphényltétracarboxylique, et leurs mélanges, présentent l'avantage de donner des (co)polyimides thermoplastiques, semi-cristallins dont la Tg est supérieure à 150°C. Ces propriétés sont également obtenues avec les esters correspondants des dianhydrides ou des tétra-acides tels que définis ci-dessus.

Cette diamine est avantageusement choisie dans le groupe constitué par :
la 1-méthyltétraméthylène 1,4-diamine, la 2-méthyltétraméthylène 1,4-diamine, la 1,1-diméthyltétraméthylène 1,4-diamine, la 1,2-diméthyltétraméthylène 1,4-diamine, la 1,3-diméthyltétraméthylène 1,4-diamine, la 1,4-diméthyltétraméthylène 1,4-diamine, la 2,2-diméthyltétraméthylène 1,4-diamine, la 2,3-diméthyltétraméthylène 1,4-diamine,
la 1-méthylpentaméthylène 1,5-diamine, la 2-méthylpentaméthylène 1,5-diamine, la 3-méthylpentaméthylène 1,5-diamine, la 1,1-diméthylpentaméthylène 1,5-diamine, la 1,2-diméthylpentaméthylène 1,5-diamine, la 1,3-diméthylpentaméthylène 1,5-diamine, la 1,4-diméthylpentaméthylène 1,5-diamine, la 1,5-diméthylpentaméthylène 1,5-diamine, la 2,2-diméthylpentaméthylène 1,5-diamine, la 3,3-diméthylpentaméthylène 1,5-diamine, la 2,3-diméthylpentaméthylène 1,5-diamine, la 2,4-diméthylpentaméthylène 1,5-diamine, la 2,5-diméthylpentaméthylène 1,5-diamine,
la 1-méthylhexaméthylène 1,6-diamine, la 2-méthylhexaméthylène 1,6-diamine, la 3-méthylhexaméthylène 1,6-diamine, la 1,1-diméthylhexaméthylène 1,6-diamine, la 1,2-diméthylhexaméthylène 1,6-diamine, la 1,3-diméthylhexaméthylène 1,6-diamine, la 1,4-diméthylhexaméthylène 1,6-diamine, la 1,5-diméthylhexaméthylène 1,6-diamine, la 1,6-diméthylhexaméthylène 1,6-diamine, la 2,2-diméthylhexaméthylène 1,6-diamine, la 3,3-diméthylhexaméthylène 1,6-diamine, la 2,3-diméthylhexaméthylène 1,6-diamine, la 2,4-diméthylhexaméthylène 1,6-diamine, la 2,5-diméthylhexaméthylène 1,6-diamine, la 2,6-diméthylhexaméthylène 1,6-diamine, la 3,4-diméthylhexaméthylène 1,6-diamine,
la 1-éthyltétraméthylène 1,4-diamine, la 2-éthyltétraméthylène 1,4-diamine, la 1,1-diéthyltétraméthylène 1,4-diamine, la 1,2-diéthyltétraméthylène 1,4-diamine, la 1,3-diéthyltétraméthylène 1,4-diamine, la 1,4-diéthyltétraméthylène 1,4-diamine, la 2,2-diéthyltétraméthylène 1,4-diamine, la 2,3-diéthyltétraméthylène 1,4-diamine,
la 1-éthylpentaméthylène 1,5-diamine, la 2-éthylpentaméthylène 1,5-diamine, la 3-éthylpentaméthylène 1,5-diamine, la 1,1-diéthylpentaméthylène 1,5-diamine, la 1,2-diéthylpentaméthylène 1,5-diamine, la 1,3-diéthylpentaméthylène 1,5-diamine, la 1,4-diéthylpentaméthylène 1,5-diamine, la 1,5-diéthylpentaméthylène 1,5-diamine, la 2,2-diéthylpentaméthylène 1,5-diamine, la 3,3-diéthylpentaméthylène 1,5-diamine, la 2,3-diéthylpentaméthylène 1,5-diamine, la 2,4-diéthylpentaméthylène 1,5-diamine, la 2,5-diéthylpentaméthylène 1,5-diamine,
la 1-éthylhexaméthylène 1,6-diamine, la 2-éthylhexaméthylène 1,6-diamine, la 3-éthylhexaméthylène 1,6-diamine, la 1,1-diéthylhexaméthylène 1,6-diamine, la 1,2-diéthylhexaméthylène 1,6-diamine, la 1,3-diéthylhexaméthylène 1,6-diamine, la 1,4-diéthylhexaméthylène 1,6-diamine, la 1,5-diéthylhexaméthylène 1,6-diamine, la 1,6-diéthylhexaméthylène 1,6-diamine, la 2,2-diéthylhexaméthylène 1,6-diamine, la 3,3-diéthylhexaméthylène 1,6-diamine, la 2,3-diéthylhexaméthylène 1,6-diamine, la 2,4-diéthylhexaméthylène 1,6-diamine, la 2,5-diéthylhexaméthylène 1,6-diamine, la 2,6-diéthylhexaméthylène 1,6-diamine, la 3,4-diéthylhexaméthylène 1,6-diamine,
la 1-éthyl-2-méthyltétraméthylène 1,4-diamine, la 1-méthyl-2-éthyltétraméthylène 1,4-diamine, la 1-méthyl-3-éthyltétraméthylène 1,4-diamine, la 1-éthyl-3-méthyltétraméthylène 1,4-diamine, la 1-méthyl-4-éthyltétraméthylène 1,4-diamine, la 1-méthyl-1-éthyltétraméthylène 1,4-diamine, la 2-méthyl-2-éthyltétraméthylène 1,4-diamine,
la 1-éthyl-2-méthylpentaméthylène 1,5-diamine, la 1-méthyl-2-éthylpentaméthylène 1,5-diamine, la 1-méthyl-3-éthylpentaméthylène 1,5-diamine, la 1-éthyl-3-méthylpentaméthylène 1,5-diamine, la 1-méthyl-4-éthylpentaméthylène 1,5-diamine, la 1-éthyl-4-méthylpentaméthylène 1,5-diamine, la 1-méthyl-5-éthylpentaméthylène 1,5-diamine, la 1-méthyl-1-éthylpentaméthylène 1,5-diamine, la 2-méthyl-2-éthylpentaméthylène 1,5-diamine, la 3-méthyl-3-éthylpentaméthylène 1,5-diamine,
la 1-éthyl-2-méthylhexaméthylène 1,6-diamine, la 1-méthyl-2-éthylhexaméthylène 1,6-diamine, la 1-méthyl-3-éthylhexaméthylène 1,6-diamine, la 1-éthyl-3-méthylhexaméthylène 1,6-diamine, la 1-méthyl-4-éthylhexaméthylène 1,6-diamine, la 1-éthyl-4-méthylhexaméthylène 1,6-diamine, la 1-méthyl-5-éthylhexaméthylène 1,6-diamine, la 1-éthyl-5-méthylhexaméthylène 1,6-diamine, la 1-méthyl-6-éthylhexaméthylène 1,6-diamine, la 1-méthyl-1-éthylhexaméthylène 1,6-diamine, la 2-méthyl-2-éthylhexaméthylène 1,6-diamine, la 3-méthyl-3-éthylhexaméthylène 1,6-diamine,
et leurs mélanges

Dans un mode avantageux de réalisation de l'invention, la diamine de formule I est choisie parmi la 1,1-diméthyltétraméthylène 1,4-diamine, la 1,2-diméthyltétraméthylène 1,4-diamine, la 1,3-diméthyltétraméthylène 1,4-diamine, la 1,4-diméthyltétraméthylène 1,4-diamine, la 2,2-diméthyltétraméthylène 1,4-diamine, la 2,3-diméthyltétraméthylène 1,4-diamine, 1-méthylpentaméthylène 1,5-diamine, la 2-méthylpentaméthylène 1,5-diamine, la 3-méthylpentaméthylène 1,5-diamine, la 1-éthyltétraméthylène 1,4-diamine, la 2-éthyltétraméthylène 1,4-diamine, et leurs mélanges.

Encore plus préférentiellement, la diamine de formule I est la 2-éthyltétraméthylène 1,4-diamine, la 2-méthylpentaméthylène 1,5-diamine ou un mélange de celles-ci.

De préférence, la diamine de formule I est la 2-méthylpentaméthylène 1,5-diamine (RN CAS : 15520-10-2).

Le (co)polyimide peut comprendre au moins une autre diamine aliphatique de formule (II) NH₂-R'-NH₂ dans laquelle R' est un radical divalent hydrocarboné, aliphatique ou cycloaliphatique, ou arylaliphatique saturé ou insaturé, et comprenant éventuellement des hétéroatomes.

Comme expliqué plus haut, les diamines de formule II de l'invention sont dites aliphatiques dans la mesure où les fonctions amines ne sont pas liées de manière covalente à un atome de carbone d'un groupement aromatique comme un phényle par exemple. C'est le cas par exemple de la méta-xylylène diamine ou la para-xylylène diamine.

En d'autres termes, R' ne comprend pas de fonction amine primaire directement liée à un cycle aromatique.

Le radical R' comprend généralement de 2 à 100 atomes de carbone, préférentiellement de 4 à 50 atomes de carbone. Le radical R' peut éventuellement contenir un ou plusieurs hétéroatomes, tel que O, N, P ou S. le radical R' peut comprendre un ou plusieurs groupes fonctionnels comme des fonctions hydroxyles, sulfones, cétones, éthers, amines secondaires, amines tertiaires ou autres.

Les diamines peuvent notamment être des diamines en positions α,ω contenant de 4 à 20 groupes méthylènes.

Les diamines aliphatiques peuvent par exemple être choisies dans le groupe comprenant : le 1,2-diaminoéthane, le 1,3-diaminopropane, le 1,4-diaminobutane, le 1,5-diaminopentane, l'hexaméthylène diamine, la 2,2,4- et 2,4,4-triméthyl-hexaméthylène diamine, le 1,7-diaminoheptane, le 1,8-diaminooctane, la 2-méthyl-1,8-diaminooctane, la 2,2,7,7-tétraméthyl-octaméthylène diamine, le 1,9-diaminonane, la 5-méthyl-1,9-diaminononane, le 1,10-diaminodécane, le 1,11-diaminoundécane, le 1,12-diaminododécane, le 1,13-diaminotridécane, le 1,14-diaminotétradécane, les diamines issues de dimères d'acide gras en C36 connus par exemple sous le nom PRIAMINE™ (référence 1075) commercialisée par CRODA.

Les diamines cycloaliphatiques sont par exemple choisies dans le groupe comprenant l'isophorone diamine, le 1,3-diaminocyclohexane, le 1,4-diaminocyclohexane, et le diaminodicyclohexyl-méthane.

On peut citer des exemples de diamines contenant des hétéroatomes comme les polyétherdiamines telles que les Jeffamine® et Elastamine® commercialisées par Hunstman. Il existe une variété de polyéther, composés de motifs oxyde d'éthylène, oxyde de propylène, oxyde de tétraméthylène. La masse molaire moyenne en nombre Mn est comprise entre 100 et 5000 g/mol.

Avantageusement, lorsque l'on utilise un mélange de plusieurs diamines aliphatiques (b), la 2-méthylpentaméthylène 1,5-diamine représente au moins 60% molaire par rapport à l'ensemble des diamines (b) mises en oeuvre, de préférence au moins 80% molaire. Selon un mode particulièrement préféré, la 2-méthylpentaméthylène 1,5-diamine est utilisée comme seule diamine (b) selon l'invention.
Selon un mode particulier de réalisation de l'invention, le (co)polyimide selon l'invention est obtenu par polymérisation d'anhydride pyromellitique et de 2-méthylpentaméthylène 1,5-diamine. Le polyimide ainsi obtenu est particulièrement avantageux puisqu'il est semi-cristallin et qu'il présente un compromis Tf/Tg intéressant, les deux températures étant suffisamment élevées tout en permettant au polyimide d'être transformé par les procédés de mise en forme des polymères thermoplastiques.

Selon un deuxième mode de réalisation de l'invention, le (co)polyimide selon l'invention est obtenu par polymérisation d'acide pyromellitique, de 2-méthylpentaméthylène 1,5-diamine et d'hexaméthylène 1,6-diamine.

Selon un troisième mode de réalisation de l'invention, le (co)polyimide selon l'invention est obtenu par polymérisation d'acide pyromellitique, de 2-méthylpentaméthylène 1,5-diamine et de 1,10-décanediamine.

### Les sels

La présente invention concerne aussi un sel de carboxylate d'ammonium obtenu à partir d'un ou plusieurs composés (a) et d'une ou plusieurs diamines aliphatiques (b) telles que définies ci-dessus.

Le sel selon l'invention peut comprendre en outre au moins un composé limiteur de chaîne choisi parmi les monoamines, les monoacides ou les diacides en positions α,β tels qu'ils peuvent former une fonction anhydride par réaction de déshydratation.
Avantageusement, le composé de formule IV est choisi parmi : le 1-aminopentane, le 1-aminohexane, le 1-aminoheptane, le 1-aminooctane, le 1-aminononane, le 1-aminodécane, le 1-aminoundécane, le 1-aminododécane, la benzylamine, l'acide ortho-phtalique (ou acide 1,2-benzènedicarboxylique), l'acide acétique, l'acide propionique, l'acide benzoïque, l'acide stéarique ou leurs mélanges.
On préfère le 1-aminohexane, le 1-aminododécane, la benzylamine ou leurs mélanges. Ces composés ont pour effet de limiter la masse molaire du (co)polyimide et ainsi de limiter la viscosité fondu du c(o)polyimide, ce qui le rend plus aisément transformable par refusion pour réaliser des articles.

La teneur en limiteur de chaîne peut aller de 0,1 à 10 % en nombre de moles, notamment de 1 à 5% en nombre de moles, par rapport au nombre total de moles de monomères, c'est à dire acide tétracarboxylique, diamine et limiteur de chaine.

### Les procédés de synthèse

Plusieurs procédés de fabrication des (co)polyimides selon l'invention par polymérisation des monomères (a) et (b) sont possibles.
Il est par exemple possible de réaliser une polymérisation en solution (ou voie solvant), notamment en suivant les voies traditionnelles de synthèse des polyimides en solvant, par exemple en 2 étapes en passant par l'intermédiaire d'un polyacide amique (PAM).
On peut également effectuer une polymérisation à l'état fondu ou à l'état solide, de mélanges de monomères ou à partir de sels précurseurs de ces monomères.
De préférence, on choisira une synthèse en voie solide, telle que décrite ci-après. Enfin, une autre technique décrite ci-après sous le nom de « spray drying » peut s'avérer aussi très avantageuse pour la fabrication des (co)polyimides selon l'invention.

### Synthèse en solution (passage par PAM)

Le procédé de synthèse des (co)polyimides en voie solvant est un procédé en deux étapes qui consiste à faire réagir dans une première étape dans un solvant aprotique polaire, comme le diméthyle acétamide, le diméthyle formamide, les crésols ou encore dans la N-méthylpyrrolidone, un dianhydride aromatique avec une diamine pour former un intermédiaire appelé polyacide amique, qui est ensuite transformé en (co)polyimide dans une seconde étape par élévation de la température ou par une déshydratation chimique.

Lors de la première étape, les amines ouvrent les cycles anhydride et donnent lieu à une fonction amide acide appelée souvent acide amique. Le polyacide amique formé est généralement soluble dans le solvant de synthèse et est transformé par cyclisation en (co)polyimide qui le plus souvent est insoluble.

Le contrôle de la masse molaire moyenne en nombre peut être obtenu :
- par l'utilisation de limiteurs de chaînes, c'est-à-dire des molécules choisies parmi les monoamines, les monoanhydrides, les monoacides ou les diacides en positions α,β tels qu'ils peuvent former une fonction anhydride par réaction de déshydratation, parmi les limiteurs de chaîne on peut citer l'anhydride phtalique, le 1-aminopentane, le 1-aminohexane, le 1-aminoheptane, le 1-aminooctane, le 1-aminononane, le 1-aminodécane, le 1-aminoundécane, le 1-aminododécane, la benzylamine, l'acide ortho-phtalique (ou acide 1,2-benzènedicarboxylique), l'acide acétique, l'acide propionique, l'acide benzoïque, l'acide stéarique ou leurs mélanges,
- par un déséquilibre stoechiométrique r=[composés aromatiques (a)]/[diamines (b)],
- par l'utilisation d'agents de branchement, c'est-à-dire des molécules de fonctionnalité supérieure à 3,
- par l'ajustement des conditions opératoires de synthèses telles que le temps de séjour, la température, l'humidité ou la pression, ou
- par une combinaison de ces différents moyens.

Le contrôle de la stoechiométrie peut être fait à tout moment du procédé de fabrication.

En particulier le déséquilibre stoechiométrique r peut aller de 0,8 à 1,2.

La teneur en limiteur de chaîne peut aller de 0,1 à 10 % en nombre de moles, notamment de 1 à 5% en nombre de moles, par rapport au nombre total de moles de monomères, c'est à dire dianhydride tétracarboxylique, diamine et limiteur de chaine.

Dans ce procédé, des catalyseurs, des charges inorganiques inertes ou réactives (argiles, silices ou précurseurs de silice, nanoparticules, ...), des stabilisants, des mâtifiants ou des colorants peuvent également être introduits.

Par exemple, pour faire un film en (co)polyimide, il est possible de verser une solution de polyacide amique sur une surface chauffante. Lors du chauffage de la surface chauffante, le solvant s'évapore et la cyclisation se fait permettant l'obtention d'un film de (co)polyimide.

Le (co)polyimide peut être soluble ou non dans le solvant. Si le (co)polyimide n'est pas soluble dans le solvant, le (co)polyimide peut être obtenu par chauffage de la solution de polyacide amique et précipiter. Il peut ainsi être récupéré par filtration et séchage : une poudre est obtenue. Si le (co)polyimide est soluble dans le solvant, il peut être récupéré sous la forme de poudre par précipitation dans ou avec un non solvant.

### Synthèse par voie fondu

Les synthèses par voie fondu impliquent que les monomères ou précurseurs sont portés à une température :
- supérieure à la température de fusion du (co)polyimide si le (co)polyimide est semi-cristallin ou
- supérieure à la température de transition vitreuse si le (co)polyimide est amorphe.
La polymérisation à l'état fondu peut être effectuée à partir :
∘ d'une diamine et d'un dianhydride ou de ses dérivés tétraacide ou diester ou triester ou tétraester
∘ d'un sel de diamine et d'un tétraacide ou d'un diester

Avantageusement, la polymérisation en voie fondu est effectuée à partir des sels qui présente l'avantage de contrôler finement la stoechiométrie.

La réaction peut être conduite dans un réacteur de synthèse ou dans une extrudeuse munie de système de dégazage des vapeurs.

Des polymérisations à l'état fondu sont notamment décrites le brevet US2710853 à partir de diamine aliphatique et d'anhydride pyromellitique ou de dérivés diester diacide d'anhydride pyromellitique.

Le contrôle de la masse molaire moyenne en nombre peut être obtenu :
- par l'utilisation de limiteurs de chaînes, c'est-à-dire des molécules choisies parmi les monoamines, les monoanhydrides, les monoacides ou les diacides en positions α,β tels qu'ils peuvent former une fonction anhydride par réaction de déshydratation, parmi les limiteurs de chaîne on peut citer l'anhydride phtalique, le 1-aminopentane, le 1-aminohexane, le 1-aminoheptane, le 1-aminooctane, le 1-aminononane, le 1-aminodécane, le 1-aminoundécane, le 1-aminododécane, la benzylamine, l'acide ortho-phtalique (ou acide 1,2-benzènedicarboxylique), l'acide acétique, l'acide propionique, l'acide benzoïque, l'acide stéarique ou leurs mélanges,
- par un déséquilibre stoechiométrique r=[composés aromatiques (a)]/[diamines (b)],
- par l'utilisation d'agents de branchement, c'est-à-dire des molécules de fonctionnalité supérieure à 3,
- par l'ajustement des conditions opératoires de synthèses telles que le temps de séjour, la température, l'humidité ou la pression, ou
- par une combinaison de ces différents moyens.

Le contrôle de la stoechiométrie peut être fait à tout moment du procédé de fabrication.

En particulier le déséquilibre stoechiométrique r peut aller de 1,01 à 1,2. Cette plage est avantageuse car elle permet d'éviter la formation de gels par réticulation de l'amine.

La teneur en limiteur de chaîne peut aller de 0,1 à 10 % en nombre de moles, notamment de 1 à 5% en nombre de moles, par rapport au nombre total de moles de monomères, c'est à dire acide/dianydride tétracarboxylique, diamine et limiteur de chaine.

Dans ce procédé, des catalyseurs, des charges inorganiques inertes ou réactives (argiles, silices ou précurseurs de silice, nanoparticules, ...), des stabilisants, des mâtifiants ou des colorants peuvent également être introduits.

### Synthèse par voie solide

Le principe de la synthèse en voie solide consiste à préparer le (co)polyimide à une température inférieure à la température de fusion du (co)polyimide à partir d'un précurseur ; T < Tf(Pl).
Il vient d'être mis en évidence par la demanderesse une nouvelle voie de préparation industrielle et efficace des (co)polyimides semi-aromatiques.
Cette synthèse est rendue possible par l'utilisation d'une polymérisation à l'état solide d'un sel solide de carboxylate d'ammonium formé à partir d'une diamine aliphatique et d'un acide tétracarboxylique aromatique. Les (co)polyimides obtenus sont des thermoplastiques généralement semi-cristallins et ont comme propriété de ne pas libérer ou absorber de l'eau lors des étapes ultérieures de transformation comme par exemple la pultrusion, l'extrusion, ou le moulage par injection. Le procédé de l'invention permet l'obtention de poudres de granulométries contrôlées puisque la réaction de polymérisation se produit à l'état solide.

Par ailleurs, la polymérisation à l'état solide permet d'éviter l'utilisation de solvants cancérigènes ou néfastes pour l'environnement.

Un autre avantage du procédé de l'invention est la faculté d'effectuer une polymérisation à une température relativement faible permettant d'éviter des dégradations thermiques du sel et du (co)polyimide formé.

La présente invention concerne ainsi un procédé de préparation de (co)polyimides selon l'invention comprenant au moins les étapes suivantes :
(a) on place dans un réacteur un sel formé par une réaction entre au moins une diamine aliphatique et au moins un acide tétracarboxylique aromatique ;
(b) on effectue une polymérisation à l'état solide à partir du sel de l'étape (a) pour obtenir le (co)polyimide à une pression absolue comprise entre 0,005 et 1 MPa et à une température T obéissant à la relation suivante :
   Tf du (co)polyimide à obtenir > T
      préférentiellement Tf du (co)polyimide à obtenir > T > Tg
      et encore plus préférentiellement
   Tf du sel de l'étape (a) > T > Tg du (co)polyimide à obtenir,
      et
(c) on récupère les particules solides (co)polyimide.

### Etape (a)

Lors de l'étape (a) du procédé, on place ainsi dans un réacteur un sel formé par une réaction entre au moins une diamine aliphatique et au moins un acide tétracarboxylique.

Un tel sel peut être synthétisé de diverses manières, connues de l'homme du métier.

On peut par exemple procéder à une addition d'une diamine aliphatique dans une solution comprenant l'acide tétracarboxylique. On peut également dissoudre l'acide tétracarboxylique dans un solvant tel que de l'alcool, comme l'éthanol ou du méthanol par exemple, et faire de même pour la diamine aliphatique. Ces deux solutions sont alors mélangées sous agitation. Le sel de carboxylate d'ammonium formé peut être insoluble dans le solvant utilisé et ainsi précipiter. Le sel peut alors être récupéré par filtration, lavé et séché et éventuellement broyé.

On peut également réaliser une solution de sel de carboxylate d'ammonium puis la concentrer à chaud et ensuite la refroidir. Le sel cristallise alors et les cristaux sont récupérés et séchés. La concentration de la solution peut être obtenue par évaporation du solvant comme l'eau ou l'alcool ou selon un autre procédé par addition d'acide tétracarboxylique et/ou de diamine aliphatique. On peut également procéder à une saturation de la solution, c'est-à-dire effectuer un procédé qui permet de modifier la concentration du sel dans la solution à une valeur compatible avec une cristallisation de celui-ci. Généralement cette concentration est au moins égale et plus préférentiellement supérieure à la concentration de saturation du sel à la température considérée. Plus précisément, cette concentration correspond à une sursaturation de la solution du sel. On peut également travailler à une pression permettant d'évaporer le solvant de la solution, tel que l'eau ou l'alcool, pour saturer la solution et provoquer la cristallisation. On peut aussi saturer la solution par addition successive ou simultanée d'un flux d'acide tétracarboxylique et d'un flux de diamine dans une solution de sel.

A titre d'exemple, on dissout l'acide tétracarboxylique dans de l'alcool, comme l'éthanol par exemple, dans un premier milieu. On dissout la diamine aliphatique dans de l'alcool dans un autre milieu et on mélange ensuite les deux milieux sous agitation. Le sel obtenu précipite.

A la fin de cette synthèse le sel peut être sous forme de poudre sèche, sous forme de poudre dispersée dans un solvant, ou dissout en solution. On peut récupérer le sel par filtration dans le cas d'un précipitât et désagréger le gâteau de filtration si nécessaire. Dans le cas ou le sel est dissout en solution, on peut le récupérer par un procédé de cristallisation par concentration, sursaturation ou en le faisant précipiter par addition d'un non solvant. Le sel cristallisé peut alors être récupéré par filtration et le gâteau de filtration peut être désagrégé si nécessaire. Un autre procédé permettant de récupérer les particules dispersées de sel sec est l'atomisation de la solution, c'est-à-dire notamment une opération d'évaporation soudaine du solvant pulvérisé sous forme de fines gouttelettes afin de récupérer les particules dispersées de sel.

Il est enfin possible de cribler la dimension des particules de sel, par exemple par tamisage ou broyage.

### Etape (b)

Lors de l'étape (b) du procédé, on effectue ainsi une polymérisation à l'état solide à partir du sel de l'étape (a) pour obtenir le (co)polyimide (I) à une pression absolue comprise entre 0,005 et 1 MPa et à une température T obéissant à la relation telle que décrite précédemment.

La pression absolue lors de l'étape (b) est préférentiellement comprise entre 0,005 MPa et 0,2 MPa.

La température lors de l'étape (b) est préférentiellement comprise entre 100°C et 250°C.

Le procédé de polymérisation à l'état solide peut être réalisé selon les procédés classiques connus de l'homme du métier. Le principe fondamental de ces procédés consiste à porter le sel de départ, sous air ou dans une atmosphère inerte ou sous vide, à une température inférieure à son point de fusion mais suffisante pour permettre la réaction de polymérisation, généralement supérieure à la température de transition vitreuse du (co)polyimide. Un tel procédé peut donc comprendre brièvement :
a) un chauffage du produit par diffusion conductive, convective ou par radiation,
b) un inertage par application de vide, balayage par un gaz neutre tel que l'azote, le CO₂, ou la vapeur surchauffée, ou application d'une surpression,
c) une élimination du sous-produit de condensation par évaporation puis, balayage du gaz vecteur ou concentration de la phase gaz,
d) une agitation mécanique ou fluidisation de la phase solide par le gaz vecteur ou vibrations peut être souhaitable afin d'améliorer les transferts thermiques et massiques et également prévenir tout risque d'agglomération du solide divisé.

Préférentiellement, on utilise dans l'étape b) un moyen de maintien en mouvement des particules de sel de (co)polyimides afin de prévenir une agrégation de ces particules. On peut utiliser pour ce faire une agitation mécanique, tel qu'un agitateur, une mise en rotation du réacteur, ou une agitation par vibrations, ou une fluidification par un gaz vecteur.

La masse molaire moyenne en nombre Mn des (co)polyimides peut être comprise entre 500 g/mol à 50000 g/mol.

Le contrôle de la masse molaire moyenne en nombre peut être obtenu :
- par l'utilisation de limiteurs de chaînes, c'est-à-dire des molécules choisies parmi les monoamines, les monoanhydrides les monoacides ou les diacides en positions α,β tels qu'ils peuvent former une fonction anhydride par réaction de déshydratation. Des exemples de limiteurs de chaîne sont l'anhydride phtalique, le 1-aminopentane, le 1-aminohexane, le 1-aminoheptane, le 1-aminooctane, le 1-aminononane, le 1-aminodécane, le 1-aminoundécane, le 1-aminododécane, la benzylamine, l'acide ortho-phtalique (ou acide 1,2-benzènedicarboxylique), l'acide acétique, l'acide propionique, l'acide benzoïque, l'acide stéarique ou leurs mélanges,
- par un déséquilibre stoechiométrique r=[composés aromatiques (a)]/[diamines (b)]
- par l'utilisation d'agents de branchement, c'est-à-dire des molécules de fonctionnalité supérieure à 3,
- par l'ajustement des conditions opératoires de synthèses telles que le temps de séjour, la température, l'humidité ou la pression, ou
- par une combinaison de ces différents moyens.

Le contrôle de la stoechiométrie peut être fait à n'importe quel moment du procédé de fabrication.

En particulier le déséquilibre stoechiométrique r peut aller de 1,01 à 1,2.

Selon un mode de réalisation particulier, le sel est :
- additionné de limiteurs de chaînes et/ou
- additionné d'un excès d'un des monomères, afin de créer un déséquilibre stoechiométrique, c'est-à-dire afin que r soit différent de 1.

Selon une variante, le limiteur de chaîne est additionné au sel de l'étape (a) déjà formé.

Selon une autre variante, le limiteur de chaîne est également sous forme de sel, en particulier il forme un sel avec la diamine aliphatique et/ou avec l'acide tétracarboxylique. En particulier le limiteur de chaine est présent lors de la formation du sel de l'étape (a) et est additionné en même temps que l'espèce lui correspondant, par exemple limiteur de type acide avec l'acide tétracarboxylique et limiteur de type amine avec l'amine aliphatique.
Dans ce deuxième cas, le limiteur de chaîne permet la formation de sel, et notamment peut être choisi dans les listes ci-dessus, à l'exception des anhydrides.

La teneur en limiteur de chaîne peut aller de 0,1 à 10 % en nombre de moles, notamment de 1 à 5% en nombre de moles, par rapport au nombre total de moles de monomères, c'est à dire acide tétracarboxylique, diamine et limiteur de chaine.

Lorsqu'un limiteur de chaine est utilisé, les quantités d'amines et d'acides peuvent être équilibrées, c'est-à-dire que la somme de fonctions amines est sensiblement égale à la moitié de la somme de fonctions acides avec lesquelles elles peuvent réagir. Par « sensiblement égale » on entend une différence maximum de 1 %.

Lorsqu'un limiteur de chaine est utilisé, les quantités d'amines et d'acides peuvent être déséquilibrées, c'est-à-dire que la somme de fonctions amines est sensiblement différente à la moitié de la somme de fonctions acides avec lesquelles elles peuvent réagir. Par « sensiblement différente » on entend une différence d'au moins 1 %.

Des catalyseurs peuvent également être introduits, et également des charges inorganiques inertes ou réactives (argiles, silices ou précurseurs de silice, nanoparticules, ...), des stabilisants, des mâtifiants, des colorants, etc.

On peut utiliser des catalyseurs, ajoutés à n'importe quel moment du procédé, tel que par exemple en mélange avec la diamine et/ou l'acide tétracarboxylique, en mélange au sel formé soit en solution soit par imprégnation à l'état solide.

Par ailleurs, il existe des applications pour lesquelles il est nécessaire que les polymères se présentent sous la forme de poudres. C'est le cas notamment du frittage laser ou des procédés de fabrication de composites à fibres continues à partir de poudres par poudrage d'étoffes ou pultrusion de monofil de verre ou de carbone, ou encore d'autres procédés. Les technologies de production de poudres de polymères connues nécessitent soit de dissoudre un polymère dans un solvant et de précipiter dans un non solvant ; mais ceci implique l'utilisation de solvants toxiques et cancérigènes, soit de mélanger à l'état fondu le polymère avec une espèce non miscible de sorte à générer une ségrégation du polymère souhaitée, soit de broyer des granulés de polymères formulés ce qui impose des étapes de micronisation et de séchages supplémentaires. Quel que soit le cas cité, les procédés sont complexes et coûteux.

### Synthèse par « spray drying »

Dans cette voie, une solution des monomères ou d'un sel de ces derniers dans un solvant, généralement de l'eau, est préparée. La solution est chauffée sous pression, ce qui déclenche la réaction de polymérisation. Le mélange est ensuite « flashé », c'est-à-dire qu'on fait subir au mélange un retour à une pression atmosphérique très rapide avec élimination de la vapeur d'eau, avant d'être pulvérisé via une buse. Un exemple d'un tel procédé est décrit dans le brevet US4603193. On obtient ainsi des particules de polyimide qui peuvent avantageusement être soumises à une étape supplémentaire de post-condensation en phase solide ou liquide, de sorte à augmenter la masse molaire moyenne en nombre Mn des (co)polyimides ainsi obtenus jusqu'à la valeur désirée.

### Compositions

On peut utiliser le (co)polyimide de l'invention pour faire de compositions qui sont généralement obtenues par mélange des différents composés, charges et/ou additifs. On procède à plus ou moins haute température, à plus ou moins haute force de cisaillement selon la nature des différents composés. Les composés peuvent être introduits simultanément ou successivement. On utilise généralement un dispositif d'extrusion dans lequel la matière est chauffée, puis fondue et soumise à une force de cisaillement, et véhiculée. On peut, selon des modes de réalisations particuliers, effectuer des pré-mélanges, en fondu ou non, avant préparation de la composition finale. On peut par exemple effectuer un pré-mélange dans une résine, par exemple du (co)polyimide, de façon à réaliser un mélange maitre.

L'invention concerne ainsi également un procédé de fabrication d'une composition par mélange, en fondu ou non, de particules solides de (co)polyimides selon l'invention avec des charges de renfort ou de remplissage et/ou des agents modificateurs du choc et/ou des additifs.

La composition selon l'invention peut éventuellement comprendre un ou plusieurs autres polymères tels que par exemple les polyamides, les polyesters ou les polyoléfines. Ces autres polymères représentent avantageusement moins de 40% en poids par rapport au poids de la composition.

La composition selon l'invention peut comprendre entre 20 et 90 % en poids, préférentiellement entre 20 et 70 % en poids, et plus préférentiellement entre 35 et 65% en poids de (co)polyimide selon l'invention obtenu par le procédé de polymérisation tel que décrit précédemment, par rapport au poids total de la composition.

La composition peut en outre comprendre des charges de renfort ou de remplissage. Les charges de renfort ou de remplissage sont des charges classiquement utilisées pour la réalisation de compositions thermoplastiques, notamment à base de polyamide. On peut notamment citer les charges fibreuses de renfort, telles que telles que des fibres de verre, des fibres de carbone, ou des fibres organiques, les charges non fibreuses, telles que des charges particulaires, lamellaires et/ou les nanocharges exfoliables ou non exfoliables comme l'alumine, le noir de carbone, les argiles, le phosphate de zirconium, le kaolin, le carbonate de calcium, le cuivre, les diatomées, le graphite, le mica, la silice, le dioxyde de titane, les zéolites, le talc, la wollastonite, les charges polymériques telles que, par exemple, des particules de diméthacrylates, les billes de verre ou de la poudre de verre. On préfère notamment utiliser les fibres de renfort, telles que les fibres de verre.

La composition selon l'invention peut comprendre entre 5 et 60 % en poids de charges de renfort ou de remplissage, préférentiellement entre 10 et 40 % en poids, par rapport au poids total de la composition.

La composition selon l'invention comprenant le (co)polyimide tel que défini précédemment peut comprendre au moins un agent modificateur du choc, c'est-à-dire un composé capable de modifier la résistance aux chocs d'une composition (co)polyimide. Ces composés modificateurs du choc comprennent préférentiellement des groupements fonctionnels réactifs avec le (co)polyimide. On entend selon l'invention par groupements fonctionnels réactifs avec le (co)polyimide, des groupements capables de réagir ou d'interagir chimiquement avec les fonctions résiduelles anhydride, acide ou amine du (co)polyimide, notamment par covalence, interaction ionique ou hydrogène ou liaison de van der Walls. De tels groupements réactifs permettent d'assurer une bonne dispersion des agents modificateurs de chocs dans la matrice (co)polyimide. On peut citer par exemple les fonctions anhydrides, époxydes, esters, amines, acides carboxyliques, les dérivés carboxylates ou sulfonates.

La composition selon l'invention peut en outre comprendre des additifs généralement utilisés pour la fabrication de compositions polyimides ou polyamides. Ainsi, on peut citer les lubrifiants, les agents ignifugeants, les plastifiants, les agents nucléants, les agents anti-UV, les catalyseurs, les antioxydants, les antistatiques, les colorants, les matifiants, les additifs d'aide au moulage ou autres additifs conventionnels.

Ces charges, agents de renfort de chocs et/ou additifs peuvent être ajoutés au (co)polyimide par des moyens usuels adaptés biens connus dans le domaine des plastiques techniques, tel que par exemple lors de la salification, après la salification, lors de la polymérisation à l'état solide, ou en mélange en fondu.

Les compositions (co)polyimides sont généralement obtenues par mélange des différents composés entrant dans la composition à froid ou en fondu. On procède à plus ou moins haute température, à plus ou moins haute force de cisaillement selon la nature des différents composés. Les composés peuvent être introduits simultanément ou successivement. On utilise généralement un dispositif d'extrusion dans lequel la matière est chauffée, puis fondue et soumise à une force de cisaillement, et véhiculée.

On peut mélanger tous les composés en phase fondue au cours d'une unique opération, par exemple au cours d'une opération d'extrusion. On peut par exemple procéder à un mélange de granulés des matériaux polymériques, les introduire dans le dispositif d'extrusion afin de les fondre et de les soumettre à un cisaillement plus ou moins important. On peut, selon des modes de réalisations particuliers, effectuer des pré-mélanges, en fondu ou non, de certains des composés avant préparation de la composition finale.

### Applications

Le (co)polyimide ou les diverses compositions selon l'invention peuvent être utilisées pour tout procédé de mise en forme de fabrication d'articles plastiques.

L'invention concerne ainsi également un procédé de fabrication d'article plastique mettant en oeuvre des particules solides de (co)polyimide selon l'invention. On peut citer à cette effet diverses techniques telles que le procédé de moulage, notamment le moulage par injection, l'extrusion, l'extrusion soufflage, ou encore le rotomoulage, notamment dans le domaine de l'automobile, de l'électronique, l'aéronautique et de l'électricité par exemple. Le procédé d'extrusion peut notamment être un procédé de filage ou de fabrication de films.

La présente invention concerne par exemple la fabrication d'articles de type étoffes imprégnées ou articles composites à fibres continues. Ces articles peuvent notamment être fabriqués par mise en présence d'une étoffe et des particules de (co)polyimide selon l'invention à l'état solide ou fondu. Les étoffes sont des surfaces textiles obtenues par assemblage de fils ou de fibres solidarisés par un procédé quelconque, tel que notamment collage, feutrage, tressage, tissage, tricotage. Ces étoffes sont aussi désignées comme des réseaux fibreux ou filamenteux, par exemple à base de fibres de verre, de fibres de carbone ou autres. Leur structure peut être aléatoire, unidirectionnelle (1D), ou multidirectionnelle (2D, 2,5D, 3D ou autre).

Les particules de (co)polyimides de l'invention peuvent notamment être utilisées dans des procédés de fabrication d'articles par fusion sélective de couches de poudre de polymères, notamment le prototypage rapide par frittage en phase solide à l'aide d'un laser. La fabrication par fusion sélective de couches est un procédé de fabrication d'articles consistant à déposer des couches de matériaux sous forme de poudre, à fondre de manière sélective une partie ou un domaine d'une couche, et de venir déposer une nouvelle couche de poudre et de fondre de nouveau une partie de cette couche et ainsi de suite de manière à obtenir l'objet désiré. La sélectivité de la partie de la couche à fondre est obtenue par exemple grâce à l'utilisation d'absorbeurs, d'inhibiteurs, de masques, ou à travers l'apport d'énergie focalisée, comme par exemple un rayonnement électromagnétique tel qu'un rayon laser. On préfère notamment le frittage par additivation de couches, particulièrement le prototypage rapide par frittage à l'aide d'un laser.

Le terme « et/ou » inclut les significations « et », « ou », ainsi que toutes les autres combinaisons possibles des éléments connectés à ce terme.

D'autres détails ou avantages de l'invention apparaîtront plus clairement au vu des exemples donnés ci-dessous uniquement à titre indicatif.

### PARTIE EXPERIMENTALE

### Normes de mesures :

Les températures de fusion (Tf) et de cristallisation au refroidissement (Tc) des (co)polyimides sont déterminées par calorimétrie différentielle à balayage (DSC « Differential Scanning Calorimetry »), à l'aide d'un appareil Perkin Elmer Pyris 1, à une vitesse de 10°C/min. Les Tf et Tc des (co)polyimides sont déterminées au sommet des pics de fusion et de cristallisation. La température de transition vitreuse (Tg) déterminée sur le même appareil à une vitesse de 40 °C/min (lorsque cela est possible, elle est déterminée à 10°C/min et spécifiée dans les exemples). Les mesures sont faîtes après fusion du (co)polyimide formé à T>(Tf du (co)polyimide + 20°C).

Lorsque l'on synthétise des polyimides à partir de sels, la température de fusion du sel est déterminée comme la température de fin de l'endotherme mesurée par chauffage du sel à 10°C/min.

L'Analyse Thermo-Gravimétrique (ATG) est réalisée sur un appareil Perkin-Elmer TGA7 sur un échantillon d'environ 10 mg, par chauffage à 10°C/min sous balayage d'azote jusqu'à 600°C.

L'analyse RMN du proton est effectuée sur un spectromètre Brüker AV500. L'analyse colorimétrique CIE L*a*b* est effectuée sur un chromomètre Minolta CR-310.

### Exemple 1 : préparation de Polvimide PI MPMDPMA à partir d'un tétra-acide à 200°C

Dans un réacteur de 100 mL, 2,1693 g (0,0081 mol) d'acide pyromellitique (PMA) à 94,9% (Sigma-Aldrich) sont dissous dans 70 g d'éthanol pur sous agitation et sous un léger balayage d'azote. Dans ce réacteur est ajoutée au pousse seringue en 1 heure une solution éthanolique à 5% contenant 0,9508 g (0,0081 mol) de 2-méthylpentane-1,5-diamine (MPMD) à 99%. 10 mL d'éthanol sont utilisés pour rincer le pousse seringue. Le milieu réactionnel agité est chauffé à 70°C et maintenu pendant 2h30. Le sel de polyimide formé précipite et est récupéré par évaporation totale de l'éthanol à 60°C sous pression réduite. La poudre de sel est blanche et fine. La température de fusion du sel est de 245°C.
Le sel est ensuite porté à 200°C sous balayage d'azote pendant 25 min pour obtenir le polyimide.
Le polyimide est semi-cristallin et présente une température de fusion de 338°C (enthalpie de fusion ΔHf=36J/g), une température de cristallisation de 269°C et une Tg=187°C. Comparé aux propriétés thermiques du PEEK, le polyimide Pl MPMDPMA présente une température de fusion similaire mais présente l'avantage d'avoir une température de transition vitreuse 37°C supérieure à celle du PEEK.
Il apparaît que le Pl MPMDPMA amorce sa dégradation à partir de 398°C (1% de perte de masse) et atteint 5% de perte de masse à 437°C.
Ainsi, le Pl MPMDPMA peut être transformé par refusion entre 338°C et 398°C sans dégradation massive.

### Exemple 2 : préparation de Polvimide PI MPMDPMA à partir d'un tétra-acide à 300°C

Le sel de l'exemple 1 est chauffé à 10°C/min jusqu'à 300°C sous balayage d'azote puis est refroidi immédiatement jusqu'à température ambiante.
Les propriétés thermiques du polyimide formé sont mesurées : Tf=322°C (enthalpie de fusion ΔHf=38 J/g) et Tc=236°C. Les températures Tf et Tc apparaissent plus faibles que celles mesurées sur le polyimide préparé selon l'exemple 1. On préfère donc effectuer la polymérisation à une température inférieure à la température de fusion du sel.

### Exemple 3 : préparation de Polvimide PI MPMDPMA à partir d'un diester-diacide à 200°C

On prépare un dérivé diester-diacide de l'anhydride pyromellitique à partir de la réaction de l'anhydride pyromellitique avec de l'éthanol. Dans un ballon contenant 300 mL d'éthanol (5,14 mol) absolu anhydre sont introduits 15 g (0,069 mol) d'anhydride pyromellitique (RN CAS : 89-32-7) à 99,7% fourni par Lonza. Le milieu réactionnel est porté à reflux pendant 3 heures. L'éthanol ouvre les fonctions anhydride et produit un dérivé diester-diacide de l'anhydride pyromellitique. Le produit est récupéré par évaporation de l'éthanol en excès dans un rotavapor. Une poudre blanche est obtenue. La structure chimique du diester-diacide dérivé de l'anhydride pyromellitique est confirmée par analyse RMN dans le DMSO deutéré.

On prépare un sel de MPMD et du diester-diacide dérivé de l'anhydride pyromellitique par ajout d'une solution contenant 5 mL d'éthanol absolu et 0,272 g (2,34 mmol) de 2-méthylpentane-1,5-diamine (MPMD) à 99% à une solution contenant 5 mL d'éthanol absolu et 0,729 g (2,34 mmol) du dérivé diester-diacide de l'anhydride pyromellitique préparé précédemment, à température ambiante et sous agitation. Après deux heures d'agitation à température ambiante, le précipité formé est récupéré par filtration. La poudre de sel blanche est obtenue et séchée à l'étuve à 50°C sous vide pour retirer toute trace d'éthanol résiduel.
Le sel présente une température de fusion de 216°C.

La polymérisation du sel est effectuée par chauffage du sel à 200°C sous balayage d'azote pendant environ 30 min. On obtient un polyimide ayant les propriétés thermiques suivantes : Tf=342°C (ΔHf=21 J/g), Tc=303°C et Tg=191°C. Il apparaît que la température de cristallisation est plus élevée que le polyimide de l'exemple 1, mais également une enthalpie de fusion plus faible.
On préfère avoir une enthalpie de fusion plus élevée, signifiant que le polymère est plus cristallin.

### Exemple 4 : préparation de Polvimide PI MPMDPMA à partir d'un dianhydride en solution

Dans un ballon bicol de 50 mL muni d'un réfrigérant et d'une ampoule de coulée sont introduits 1,45 g (6,6 mmol) d'anhydride pyromellitique à 99% et 20 mL de 1,3-diméthyl-2-imidazolidinone (RN CAS : 80-73-9) comme solvant aprotique polaire. Le milieu réactionnel est agité à température ambiante puis on ajoute par l'ampoule de coulée 0,774 g (6,6 mmol) de 2-méthylpentane-1,5-diamine (MPMD) à 99%. On porte ensuite le milieu réactionnel à 140°C et on maintient 1 heure à 140°C. Après refroidissement, la solution de polyacideamique formée est précipitée dans 100 mL d'éthanol. Le précipité (solide orangé) est récupéré par filtration puis porté à 200°C pendant 30 min. Le polymère obtenu est coloré jaune-orangé et présente les propriétés thermiques suivantes : Tf=339°C, Tc=301°C, Tg=191°C.

### Exemple 5 : préparation de Polvimide PI MPMDPMA en présence d'un limiteur de chaîne

Dans un réacteur de 2L, 35 g (0,1344 mol) d'acide pyromellitique (PMA) à 97,6% sont dissous dans 665 g d'éthanol pur sous agitation sous un léger balayage d'azote. Dans ce réacteur est ajoutée avec une ampoule de coulée en 1 heure une solution contenant 15,373 g (0,131 mol) de 2-méthylpentane-1,5-diamine (MPMD) à 99%, 0,7074 g (0,00692 mol) de 1-aminohexane à 99% et 200 g d'éthanol. Après introduction de la solution dans le réacteur, 20 mL d'éthanol sont utilisés pour rincer l'ampoule de coulée. Le milieu réactionnel agité est chauffé à 70°C et maintenu pendant 3h. Le sel de polyimide formé précipite et est récupéré par évaporation totale de l'éthanol à 65°C sous pression réduite. La poudre de sel est blanche et fine.
Le sel est ensuite porté à 200°C sous balayage d'azote pendant 5 h pour obtenir le polyimide. Une poudre blanche de polyimide est obtenue : la couleur de la poudre de Pl MPMDPMA est analysée. Elle a les caractéristiques colorimétriques suivantes CIE L*=94,98, a*=0,42, b*=5,02, ce qui indique que la poudre est très blanche (les poudres légèrement jaunes présentent en général un b*>10). Ce procédé de polymérisation permet d'éviter des colorations liées soit à des dégradations ou à la présence de solvant résiduels comme c'est le cas dans les polymérisations de polyimides par en voie fondue, au-delà de la température de fusion du polyimide ou par voie solvant.

La poudre de polyimide obtenue est analysée par mesure de la viscosité relative solution dans l'acide sulfurique à 96% d'une solution à 10 g/L de polyimide dans un tube Ubbelohde de diamètre 1,03 mm associé à un viscosimètre SHOTT de référence AVS350 et à une température de 25°C. La viscosité relative est de 1,77.

### Exemple 6 : étude de la stabilité à l'état fondu du polyimide de l'exemple 5

1 g du polyimide de l'exemple 5 est placé dans un tube à essai et inerté à l'azote. Le tube est placé dans un bloc chauffé à 350°C (au-dessus de la température de fusion du polyimide) pendant 30 min. Après 30 min, la viscosité relative est déterminée à 1,75, ce qui montre que la viscosité du polyimide n'a pas évoluée lors de son passage à l'état fondu pendant 30 min. Il peut donc être facilement mis en oeuvre par refusion sous la forme d'un objet à 350°C sans dégradation majeure.

### Exemple 7 : injection du PI MPMDPMA

La poudre de polyimide Pl MPMDPMA de l'exemple 5 est injectée à l'aide d'une micro-presse à injecter associée au « micro-compounder » DSM MIDI 2000 (volume 15 cm³) par refusion du polyimide à une température de 350°C (température du fourreau) et injection dans un moule régulé à 180°C pour former des barreaux de dimension 90x13x1,6 mm³. Les barreaux sont totalement opaques et très rigides. Une analyse mécanique dynamique en flexion trois points (déformation imposée de 0,01 %, fréquence 1 Hz) est effectuée sur un appareil TA Instrument RSA3. A 23°C, le module E' est égal à 3,2 GPa et la température de transition alpha est déterminée à 193°C. Un point important est que le Pl MPMDPMA conserve sa rigidité jusqu'à 193°C, puisque par exemple à 150°C E' est encore à 2,6 GPa.

Un test de brûlage par une flamme montre que les éprouvettes présentent une bonne tenue au feu.

### Exemple comparatif 8 : préparation du polvimide PI MPMDODPA à partir d'un tétra-acide 4,4-oxydiphtalic acid à 200°C

L'anhydride 4,4'-oxydiphtalique (ODPA) à 97% acheté chez Sigma-Aldrich est hydrolysé dans l'eau chaude à reflux pendant 2 heures. On récupère ainsi l'acide 4,4'-oxydiphtalique (ODA) à 100% par évaporation de l'eau et séchage.

Dans un réacteur de 100 mL, 1,54 g (0,0044 mol) d'acide 4,4'oxydiphtalique (ODA) sont dissous dans 50 g d'éthanol pur sous agitation sous un léger balayage d'azote. Dans ce réacteur est ajoutée au pousse seringue en 5 minutes une solution éthanolique à 3,3% contenant 0,51 g (0,0044 mol) de 2-méthylpentane-1,5-diamine (MPMD) à 99%. 10 mL d'éthanol sont utilisés pour rincer le pousse seringue. Le milieu réactionnel agité est chauffé à 70°C et maintenu pendant 2h30. Le sel de polyimide formé précipite et est récupéré par évaporation totale de l'éthanol à 60°C sous pression réduite (300 mbar), puis séché une nuit sous vide à 45°C. Le sel est ensuite porté à 180°C sous balayage d'azote pendant 2 heures pour obtenir le polyimide. Le polyimide est amorphe (pas de détection de température de fusion ni de température de cristallisation) et présente une température de transition vitreuse de Tg=134,8°C.

Il apparaît que le Pl MPMDODPA est amorphe et sa Tg est inférieure à 150°C. Ainsi, s'il est sollicité au-delà de sa Tg, par exemple à 200°C comme c'est le cas dans un environnement sous capot moteur dans l'automobile, il se ramollit et perd ses propriétés mécaniques et donc ne peut pas être utilisé à cette température.

### Exemple comparatif 1: préparation du polvimide PI 5PMA à partir d'un tétra-acide à 200°C

Dans un réacteur de 150 mL, 2,192 g (0,0084 mol) d'acide pyromellitique (PMA) à 97,5% (Sigma-Aldrich) sont dissous dans 70 g d'éthanol pur sous agitation et sous un léger balayage d'azote. Dans ce réacteur est ajoutée au pousse seringue en 1 heure une solution éthanolique à 5% contenant 1,0129 g (0,0086 mol) de 1,5-pentanediamine à 86,7% (l'impureté est l'eau). 10 mL d'éthanol sont utilisés pour rincer le pousse seringue. Le milieu réactionnel agité est chauffé à 75°C et maintenu pendant 2h. Le sel de polyimide 5PMA formé précipite et est récupéré par filtration sous pression vide, puis séché une nuit à 45°C sous vide. La poudre de sel est blanche et fine.

Le sel est ensuite porté à 200°C sous balayage d'azote pendant 30 min pour obtenir le polyimide. On obtient un polyimide Pl 5PMA ayant les propriétés thermiques suivantes : Tf=407°C (deltaHf=21 J/g), Tc=391°C et Tg=196°C. Ces performances sont extrêmement élevées mais il apparaît que le Pl 5PMA amorce sa dégradation à partir de 370°C (1% de perte de masse) et atteint 5% de perte de masse à 439°C. Ainsi, le Pl 5PMA amorce sa dégradation avant sa fusion : il n'est pas envisageable de le mettre en oeuvre sous la forme d'articles par refusion.

### Exemple comparatif 2 : préparation du polyimide PI 12PMA à partir d'un tétra-acide à 200°C

Dans un réacteur de 5L sont introduits 40 g (0,15mol) d'acide pyromellitique à 94,9% (Sigma-Aldrich) et 2 litres d'éthanol pur. Le milieu réactionnel est agité et chauffé à 70°C sous léger balayage d'azote. Dans un ballon de 1L, on dissout 30,5 g (0,15 mol) de 1,12-diaminododécane à 98% (TCl Europe N.V.) dans 500 mL d'éthanol pur à température ambiante. Cette solution est ensuite placée dans une ampoule de coulée raccordée au réacteur 5L et ajoutée goutte-à-goutte en 1 heure à la solution éthanolique d'acide pyromellitique. Le contact entre la diamine et l'acide pyromellitique engendre la formation d'un sel qui précipite immédiatement sous agitation vigoureuse. Le milieu réactionnel est maintenu sous agitation vigoureuse pendant 3h 30 min à 70°C et sous azote. La poudre de sel est récupérée par filtration sur Büchner et lavée à l'éthanol, puis broyée et séchée sous vide à 50°C pendant une nuit. Le rendement massique est de 95%. La poudre est blanche et fine. La température de fusion du sel est de 260°C. La poudre de sel 12PMA est placée dans un ballon cannelé fixé sur un appareil rotavapeur et mis sous léger balayage d'azote. La pression est égale à la pression atmosphérique. Le ballon est plongé dans un bain d'huile à 200°C et mis en rotation pendant 8 heures. La poudre de Pl 12PMA obtenue est blanche, parfaitement sèche. La poudre de Pl 12PMA a une température de fusion de 303°C (enthalpie de fusion deltaHf=35J/g), une température de cristallisation de 274°C et une Tg=101°C.

Ce Pl 12PMA amorce sa dégradation à 418°C (1% de perte de masse) et atteint 5% de perte de masse à 451°C. Ainsi le Pl 12PMA est un thermoplastique semi-cristallin, qui peut être mis en forme car refusion, mais dont la Tg=101°C est faible en comparaison avec des polymères de très hautes performances comme le PEEK, ce qui limite son domaine d'utilisation dans son état vitreux à moins de 100°C.

On voit ici l'intérêt des exemples de l'invention qui permettent d'avoir des polyimides semi-cristallins dont la Tg est très élevée, bien supérieure à 150°C, tout en ayant une température de fusion compatible avec les procédés de transformation des thermoplastiques sans dégradation manifeste des propriétés.

### Exemple 9 : préparation de composite PI MPMDPMA / tissu de carbone

Un lot de 150 g de polyimide Pl MPMDPMA bloqué par la 12-aminododécane de viscosité relative 1,85 préparé selon un procédé similaire à celui décrit dans l'exemple 5 est utilisé pour faire un composite thermoplastique Pl MPMDPMA / tissu de carbone.
Avant utilisation, la poudre est séchée une nuit sous vide à 90°C.

Le renfort utilisé dans cet exemple est sous forme de préformes en tissus de carbone, coupées aux dimensions requises pour la fabrication de plaques c'est-à-dire 100 x 150 mm. L'étoffe de renfort utilisée est un tissu équilibré en fibre de carbone (0°-90°) d'origine Hexcel, présentant un grammage de 200 g/m² (3K).

La réalisation des pièces composites est faite au moyen d'une presse hydraulique 100 tonnes à double plateaux contrôlée en force et équipée d'un moule à chauffage par induction (technologie RocTool®), et de moyens de refroidissement (circulation d'eau). Le moule métallique est dotée d'une empreinte de dimensions 150 mm x 150 mm.

Pour réaliser un composite contenant 55% volume de fibres de carbone avec le tissus de grammage 200 g/m² (3K), on réalise une préforme par empilement de pli de carbone, chaque pli étant poudré de façon relativement homogène avec de la poudre de polyimide. Dans l'exemple considéré, il a été utilisé 10 plis de carbone (200 g/m²). On introduit alors la préforme constituée de l'empilement de plis poudrés dans le moule.

Après introduction de la préforme et fermeture du moule sous très faible pression, la température des plateaux de la presse est alors montée à 355°C en 91 secondes. Un palier est réalisé sous très faible pression pendant 60 secondes à 355°C. A la fin du palier (60 secondes), une pression est appliquée pendant 20 secondes : 30 bars vérin. Le refroidissement est réalisé sous pression en 6 minutes et 30 secondes : démoulage des plaques à 50°C environ.
Le temps de cycle total est inférieur à 10 minutes.
Les plaques obtenues de 2,4 mm. Les articles composites selon l'invention présentent un très bon aspect de surface.

Il est possible d'obtenir des articles composites en utilisant les polyimides selon l'invention, particulièrement en procédant à des cycles de fabrication extrêmement courts.

## Revendications

1. (Co)polyimide thermoplastique, semi-aromatique obtenu par polymérisation d'unités récurrentes qui sont constituées par les composés suivants :
(a) au moins un composé aromatique comprenant 2 fonctions anhydride et/ou ses dérivés acide carboxylique et/ou ester, choisi dans le groupe constitué par: l'anhydride pyromellitique, le 3,3',4,4'-biphényltétracarboxylique dianhydride, l'acide pyromellitique, l'acide 3,3',4,4'-biphényltétracarboxylique, et leurs mélanges ; et
(b) une ou plusieurs diamines aliphatiques dont les fonctions amines ne sont pas liées de manière covalente à un atome de carbone d'un cycle aromatique,
dans lequel ladite ou lesdites diamines aliphatiques sont choisies parmi les diamines de formule (I) NH₂-R-NH₂ avec R étant un radical divalent hydrocarboné aliphatique saturé, dont les deux fonctions amines sont séparées par 4 à 6 atomes de carbone, et dont 1 ou 2 atomes d'hydrogène du radical divalent sont substitués par 1 ou 2 groupes méthyle et/ou éthyle; le (co)polyimide pouvant comprendre en outre comme autre diamine aliphatique (b) une ou plusieurs diamines de formule (II) NH₂-R'-NH₂ avec R' étant un radical divalent hydrocarboné, aliphatique, cycloaliphatique, ou arylaliphatique, saturé ou insaturé, qui comprend éventuellement des hétéroatomes;
ou au moins un sel de carboxylate d'ammonium obtenu à partir des monomères (a) et (b).

2. (Co)polyimide selon la revendication 1, **caractérisé en ce que** le composé aromatique comprenant 2 fonctions anhydride et/ou ses dérivés acide carboxylique et/ou ester est l'acide pyromellitique,.

3. (Co)polyimide selon l'une quelconque des revendications précédentes, **caractérisé en ce que** la diamine de formule I est choisie dans le groupe constitué par : la 2-éthyl tétraméthylène 1,4-diamine, la 2-méthylpentaméthylène 1,5-diamine ou un mélange de celles-ci, de préférence la 2-méthylpentaméthylène 1,5-diamine.

4. Procédé de fabrication de (co)polyimide tel que défini à l'une des revendications 1 à 3 par polymérisation en solution des monomères (a) et (b) ou sel de carboxylate d'ammonium obtenu à partir des monomères (a) et (b).

5. Procédé de fabrication de (co)polyimide tel que défini à l'une des revendications 1 à 3 par polymérisation à l'état solide des monomères (a) et (b) ou du sel de carboxylate d'ammonium obtenu à partir des monomères (a) et (b).

6. Procédé de fabrication de (co)polyimide tel que défini à l'une des revendications 1 à 3 par polymérisation à l'état fondu des monomères (a) et (b) ou du sel de carboxylate d'ammonium obtenu à partir des monomères (a) et (b).

7. Sel de carboxylate d'ammonium obtenu à partir d'un ou plusieurs composés (a) et une ou plusieurs diamines aliphatiques (b), lesdits composés (a) et lesdites diamines (b) étant tels que définis à l'une quelconque des revendications 1 à 3.

8. Sel selon la revendication 7, **caractérisé en ce qu'**il comprend en outre au moins un composé limiteur de chaîne choisi parmi les monoamines, les monoacides ou les diacides en positions α,β tels qu'ils peuvent former une fonction anhydride par réaction de déshydratation, de préférence choisi parmi : le 1-aminopentane, le 1-aminohexane, le 1-aminoheptane, le 1-aminooctane, le 1-aminononane, le 1-aminodécane, le 1-aminoundécane, le 1-aminododécane, la benzylamine, l'acide ortho-phtalique, l'acide acétique, l'acide propionique, l'acide benzoïque, l'acide stéarique ou leurs mélanges.

9. Composition comprenant le (co)polyimide tel que défini à l'une quelconque des revendications 1 à 3 et des charges et/ou additifs.

10. Particules solides de (co)polyimide à base de (co)polyimide selon l'une quelconque des revendications 1 à 3 dont le diamètre médian D50 est inférieur ou égal à 20 mm, de préférence compris entre 2 µm et 10 mm.

11. Procédé de fabrication d'article plastique mettant en oeuvre des particules solides de (co)polyimide selon la revendication 10, par refusion desdites particules puis mise en forme d'un article par extrusion, moulage ou soufflage.des particules fondues.

12. Article plastique obtenu à partir du (co)polyimide tel que défini à l'une quelconque des revendications 1 à 3 ou de la composition telle que définie à la revendication 9.

13. Article plastique selon la revendication 12, **caractérisé en ce qu'**il s'agit d'une pièce moulée, extrudée ou soufflée.

14. Article plastique selon la revendication 13, **caractérisé en ce qu'**il s'agit d'une pièce moulée par injection, d'un composite à fibres continues, d'un film, d'une fibre, d'un fil ou d'un filament.

15. Article plastique selon la revendication 13, **caractérisé en ce qu'**il s'agit d'une pièce tissée ou tricotée à partir de fibres, de fils, ou de filaments à base de (co)polyimide tel que défini à l'une quelconque des revendications 1 à 3.

## Patentansprüche

1. Teilaromatisches thermoplastisches Copolyimid, gewonnen durch Polymerisation von sich wiederholenden Einheiten, die aus folgenden Verbindungen bestehen:
(a) mindestens eine aromatische Verbindung mit zwei funktionellen Anhydridgruppen und oder ihre Carbonsäure- und/oder Esterderivate, ausgewählt aus der Gruppe bestehend aus: Pyromellitsäureanhydrid, 3,3',4,4'-Biphenyltetracarbonsäuredianhydrid, Pyromellitsäure, 3,3',4,4'-Biphenyltetracarbonsäure und ihren Mischungen; und
(b) ein oder mehrere aliphatische Diamine, deren funktionellen Amingruppen nicht kovalent an ein Kohlenstoffatom eines aromatischen Rings gebunden sind,
wobei das oder die aliphatischen Diamine aus den Diaminen mit der Formel (I) NH₂-R-NH₂ ausgewählt sind, wobei R ein gesättigter aliphatischer zweiwertiger Kohlenwasserstoffrest ist, deren beiden funktionellen Amingruppen durch 4 bis 6 Kohlenstoffatome getrennt sind, und von denen 1 oder 2 Wasserstoffatome des zweiwertigen Rests durch 1 oder 2 Methyl- und/oder Ethylgruppen substituiert sind; wobei das Copolyimid ferner als weiteres aliphatisches Diamin (b) ein oder mehrere Diamine mit der Formel (II) NH₂-R'-NH₂ umfassen kann, wobei R' ein gesättigter oder ungesättigter aliphatischer, cycloaliphatischer oder arylaliphatischer zweiwertiger Kohlenwasserstoffrest ist, der gegebenenfalls Heteroatome umfasst;
oder mindestens ein Ammoniumcarboxylatsalz, das aus den Monomeren (a) und (b) gewonnen wird.

2. Copolyimid nach Anspruch 1, **dadurch gekennzeichnet, dass** die aromatische Verbindung, die zwei funktionelle Anhydridgruppen umfasst, und/oder ihre Carbonsäure- und/oder Esterderivate Pyromellitsäure ist.

3. Copolyimide nach einem der vorhergehenden Ansprüche, **dadurch gekennzeichnet, dass** das Diamin mit der Formel I aus der Gruppe ausgewählt ist bestehend aus: 2-Ethyltetramethylen-1,4-diamin, 2-Methylpentamethylen-1,5-diamin oder eine Mischung daraus, vorzugsweise 2-Methylpentamethylen-1,5-diamin.

4. Verfahren zur Herstellung des Copolyimids nach einem der Ansprüche 1 bis 3 durch Lösungspolymerisation der Monomere (a) und (b) oder des Ammoniumcarboxylatsalzes, das aus den Monomeren (a) und (b) gewonnen wird.

5. Verfahren zur Herstellung des Copolyimids nach einem der Ansprüche 1 bis 3 durch Polymerisation der Monomere (a) und (b) oder des Ammoniumcarboxylatsalzes, das aus den Monomeren (a) und (b) gewonnen wird, im festen Zustand.

6. Verfahren zur Herstellung des Copolyimids nach einem der Ansprüche 1 bis 3 durch Polymerisation der Monomere (a) und (b) oder des Ammoniumcarboxylatsalzes, das aus den Monomeren (a) und (b) gewonnen wird, im geschmolzenen Zustand.

7. Ammoniumcarboxylatsalz, das aus einer oder mehreren Verbindungen (a) und einer oder mehreren aliphatischen Diaminen (b) gewonnen wird, wobei die Verbindungen (a) und die Diamine (b) einem der Ansprüche 1 bis 3 entsprechen.

8. Salz nach Anspruch 7, **dadurch gekennzeichnet, dass** es ferner mindestens eine Kettenbegrenzungsverbindung umfasst, die ausgewählt ist aus Monoaminen, Monosäuren oder Disäuren in der α,β-Position, sodass sie eine funktionelle Anhydridgruppe durch eine Dehydratisierungsreaktion bilden können, vorzugsweise ausgewählt aus: 1-Aminopentan, 1-Aminohexan, 1-Aminoheptan, 1-Aminooctan, 1-Aminononan, 1-Aminodecan, 1-Aminoundecan, 1-Aminododecan, Benzylamin, Orthophthalsäure, Essigsäure, Propionsäure, Benzoesäure, Stearinsäure oder ihren Mischungen.

9. Zusammensetzung, die das Copolyimid nach einem der Ansprüche 1 bis 3 und Füllstoffe und/oder Zusatzstoffe umfasst.

10. Feste Copolyimidpartikel auf der Grundlage von Copolyimid nach einem der Ansprüche 1 bis 3, deren mittlerer Durchmesser D50 kleiner gleich 20 mm ist, vorzugsweise zwischen 2 µm und 10 mm liegt.

11. Verfahren zur Herstellung eines Kunststoffartikels, bei dem feste Copolyimidpartikel nach Anspruch 10 verwendet werden, durch erneutes Aufschmelzen der Partikel und anschließende Formgebung eines Artikels durch Extrusion, Formen oder Blasformen der geschmolzenen Partikel.

12. Kunststoffartikel, der aus dem Copolyimid nach einem der Ansprüche 1 bis 3 oder der Zusammensetzung nach Anspruch 9 gewonnen wird.

13. Kunststoffartikel nach Anspruch 12, **dadurch gekennzeichnet, dass** es sich um ein Formteil, ein Extrusionsteil oder ein Blasformteil handelt.

14. Kunststoffartikel nach Anspruch 13, **dadurch gekennzeichnet, dass** es sich um ein Spritzgussteil, einen Verbundwerkstoff mit Endlosfasern, eine Folie, eine Faser, einen Faden oder ein Filament handelt.

15. Kunststoffartikel nach Anspruch 13, **dadurch gekennzeichnet, dass** es sich um ein Teil handelt, das aus Fasern, Fäden oder Filamenten auf der Basis des Copolyimids nach einem der Ansprüche 1 bis 3 gewebt oder gestrickt ist.

## Claims

1. Semi-aromatic thermoplastic (co)polyimide obtained by polymerization of repeat units which are composed of the following compounds:
(a) at least one aromatic compound comprising 2 anhydride functional groups and/or its carboxylic acid and/or ester derivatives, selected from the group consisting of: pyromellitic anhydride, 3,3',4,4'-biphenyltetracarboxylic dianhydride, pyromellitic acid, 3,3',4,4'-biphenyltetracarboxylic acid and their mixtures; and
(b) one or more aliphatic diamines, the amine functional groups of which are not covalently bonded to a carbon atom of an aromatic ring;
in which said aliphatic diamine or diamines are chosen from the diamines of formula (I) NH₂-R-NH₂ with R being a saturated aliphatic divalent hydrocarbon radical, the two amine functional groups of which are separated by 4 to 6 carbon atoms and 1 or 2 hydrogen atoms of the divalent radical of which are replaced by 1 or 2 methyl and/or ethyl groups; it being possible for the (co)polyimide to additionally comprise, as other aliphatic diamine (b), one or more diamines of formula (II) NH₂-R'-NH₂ with R' being a saturated or unsaturated and aliphatic, cycloaliphatic or arylaliphatic divalent hydrocarbon radical, which optionally comprises heteroatoms;
or at least one ammonium carboxylate salt obtained from the monomers (a) and (b).

2. (Co)polyimide according to Claim 1, **characterized in that** the aromatic compound comprising 2 anhydride functional groups and/or its carboxylic acid and/or ester derivatives is pyromellitic acid.

3. (Co)polyimide according to either one of the preceding claims, **characterized in that** the diamine of formula I is selected from the group consisting of: 2-ethyltetramethylene-1,4-diamine, 2-methylpentamethylene-1,5-diamine or a mixture of these, preferably 2-methylpentamethylene-1,5-diamine.

4. Process for the manufacture of (co)polyimide as defined in one of Claims 1 to 3 by solution polymerization of the monomers (a) and (b) or ammonium carboxylate salt obtained from the monomers (a) and (b).

5. Process for the manufacture of (co)polyimide as defined in one of Claims 1 to 3 by solid-state polymerization of the monomers (a) and (b) or of the ammonium carboxylate salt obtained from the monomers (a) and (b).

6. Process for the manufacture of (co)polyimide as defined in one of Claims 1 to 3 by melt polymerization of the monomers (a) and (b) or of the ammonium carboxylate salt obtained from the monomers (a) and (b).

7. Ammonium carboxylate salt obtained from one or more compounds (a) and one or more aliphatic diamines (b), said compounds (a) and said diamines (b) being as defined in any one of Claims 1 to 3.

8. Salt according to Claim 7, **characterized in that** it additionally comprises at least one chain-limiting compound chosen from monoamines, monoacids or diacids in the α,β positions such that they can form an anhydride functional group by a dehydration reaction, preferably chosen from: 1-aminopentane, 1-aminohexane, 1-aminoheptane, 1-aminooctane, 1-aminononane, 1-aminodecane, 1-aminoundecane, 1-aminododecane, benzylamine, ortho-phthalic acid, acetic acid, propionic acid, benzoic acid, stearic acid or their mixtures.

9. Composition comprising the (co)polyimide as defined in any one of Claims 1 to 3 and fillers and/or additives.

10. Solid (co)polyimide particles based on (co)polyimide according to any one of Claims 1 to 3, the median diameter D50 of which is less than or equal to 20 mm, preferably between 2 µm and 10 mm.

11. Process for the manufacture of a plastic article employing solid (co)polyimide particles according to Claim 10, by remelting said particles and then shaping an article by extrusion, molding or blow molding of the molten particles.

12. Plastic article obtained from the (co)polyimide as defined in any one of Claims 1 to 3 or from the composition as defined in Claim 9.

13. Plastic article according to Claim 12, **characterized in that** it is a molded, extruded or blow-molded part.

14. Plastic article according to Claim 13, **characterized in that** it is an injection-molded part, a continuous fiber composite, a film, a fiber, a yarn or a filament.

15. Plastic article according to Claim 13, **characterized in that** it is a part woven or knitted from fibers, yarns or filaments based on (co)polyimide as defined in any one of Claims 1 to 3.
